# EUROPEAN PATENT APPLICATION

(11) **EP 0 966 934 A1**
(43) Date of publication of application: **29.12.1999**
(21) Application number: 98124718.2
(22) Date of filing: 28.12.1998
(51) Int. Cl.: A61F 5/443

(54) **Adhesive urine collector**

(30) Priority: 26.06.1998 WO PCT/US98/13289; 26.06.1998 WO PCT/US98/13288
(71) Applicant: THE PROCTER & GAMBLE COMPANY, Cincinnati, Ohio 45202 (US)
(72) Inventor: D'Acchioli, Vincenzo, 65779 Kelkheim am Taunus (DE); Palumbo, Gianfranco, 61348 Bad Homburg (DE); Schmidt, Gesche, 65779 Kelkheim am Taunus (DE); Scholz, Manuela, 60599 Frankfurt (DE)
(74) Representative: Kohol, Sonia

(57) **Abstract**

The present invention relates to urine management devices for babies, children or adults, male or female, to be adhesively attached in a releasable manner to the uro-genital area of the wearer, said devices being particularly easy to put in place and particularly easy to detach after use. Claimed and described is a urine management device (10) comprising a bag (11), said bag (11) having an aperture (21) and a flange (12) surrounding said aperture (21) said flange (12) having a wearer facing portion (23) and a garment facing portion (22), wherein said wearer facing portion (23) comprises a layer of adhesive (20) for releasable attachment to the uro-genital area of a wearer, characterised in that said flange (12) is provided with at least one non-adhesive lobe (13).

## Description

### Field of the invention

The present invention relates to urine management devices for babies, children or adults to be adhesively attached in a releasable manner to the uro-genital area of the wearer, said devices being particularly easy to put in place and particularly easy to detach after use.

### Background

Urine management devices are known articles of manufacture that are designed to be worn principally by incontinence sufferers and infants. Such urine management devices are attached to the uro-genital region of the wearer and are intended to entrap and immediately contain urine and other bodily discharges. As a consequence, these devices are functionally effective in lessening epidermal irritation; in preventing contamination of articles such as clothing and bedding; and even in preventing the soiling of the carers themselves.

Typically, the urine management devices are made from a plastic material. For instance, GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material. GB 2,268,882 discloses a urostomy pouch/bag of plastic material provided with a stomal orifice which is surrounded by a first coupling member, by which the pouch can be affixed to a counterpart coupling member, which can be attached to a wearer. The pouch may also be provided for use as a kit further comprising an applicator of super absorbent material which can be injected into the pouch by the use of a plunger. US 4,804,377 discloses a collector for urine specimens from children. The collector comprises a rectangular flange for adhesive attachment, which comprises a notch to facilitate easy access to the garment facing side of the flange. EP 140 478 discloses a disposable diaper having a water proof barrier preferably polypropylene or polyethylene formed as a flattened bag having a single opening. The bag is provided with two layers filler materials, the upper layer being a wicking material and the lower being a super absorbent material. WO 85/03428 discloses a urine retaining device for male patients comprising a protecting bag having an opening, parallelogram-walls of a water impermeable laminate of polyethylene film and nonwoven provided with an insert of water absorbing material.

Besides and in connection with optimum adherence, the proper placement of the device is a key issue in the field of urine management devices. Total or substantial misplacement of the device will lead to a severe misfunctioning, in particular incomplete collection of urine and leaking. If the aperture of the urine management device is not sufficiently in registry with the urinary duct of a wearer, substantial pressure, in particular on the flanges of the device, can build up in the urination process and lead to the detachment of the device. Also the adhesive on the flange may come into contact with urine to an undesirable extent. With some adhesives this can lead to the detachment of the adhesively secured device, again obviously entailing the most unwanted consequences.

If the misplacement of the device is recognised before use, the placement of the device is normally corrected. The necessary detachment and reattachment of the device means an additional stress on the skin of the wearer. Many wearers, who make use of urine management devices have a sensitive skin due to their age, whether very old or very young, and furthermore sometimes also suffer from skin irritations. Proper placement of the device in the first place is therefore highly desirable.

The urine management devices which are disclosed in the mentioned prior art are normally handled and placed onto the skin of the wearer by using the flange itself or the portions of the bag, such as a neck portion. One of the first necessary handling steps is to remove the release paper from the adhesive surface of the flange. When then placing the device, the caretaker or wearer may touch the adhesive area of the flange with the fingers and leave finger marks. Such marks will reduce the adhesive force of the affected areas, if dirt is deposited from the fingers or if an adhesive is used, which tends to adhere less on a second contact with a surface.

Furthermore, during application of the urine management device to the wearer by holding the flange, pressure typically needs to be exercised upon the flange. However, as a result the flange may suffer deformation, such deformation leading to a poorer performance of the device, in particular to a poorer adhesion, discomfort or possibly leaking of the device.

Yet another problem associated with urine management devices is their handling after detachment. Since they regularly are a source of malodour and possibly of leakage, the caretaker will typically try and seal the bag for disposal, e.g. by sticking opposite parts of the adhesive flange together. In doing so the caretaker might face the problem of unwanted sticking of the fingers onto the adhesive flange or between two parts of the adhesive flange. Also, the caretaker might not want to touch the adhesive parts of the flange, since they get easily soiled due to their adhesive nature.

In attempting to overcome all the aforementioned problems relating to the prior art, it has now been found that adhesive urine management devices can be designed which have excellent ease of placement properties, through the use of a simple, but efficient device. The same design does not only greatly help in placing the device but also assists in detachment and handling after detachment.

### Brief summary of the invention

This invention relates to a urine management device (10) comprising a bag (11) and a flange (12). The flange (12) comprises adhesive used to attach the device to the uro-genital area of the wearer. The invention resides principally in the provision of at least one non-adhesive lobe on the flange (12). Said lobes (13) can be used by a caretaker to handle the device. Said lobes (13) are also useful for the detachment of the device and as a help in the peeling off of the release paper.

### Brief description of the drawings

Figure 1 is a top plan view of a preferred embodiment of the urine management device comprising two lobes. L denotes a longitudinal axis, T denotes a transversal axis.
Figure 2 is a top plan view of another preferred embodiment of the urine management device comprising two lobes.
Figure 3 is a top plan view of another preferred embodiment of the urine management device comprising one lobe.

### Detailed description of the invention

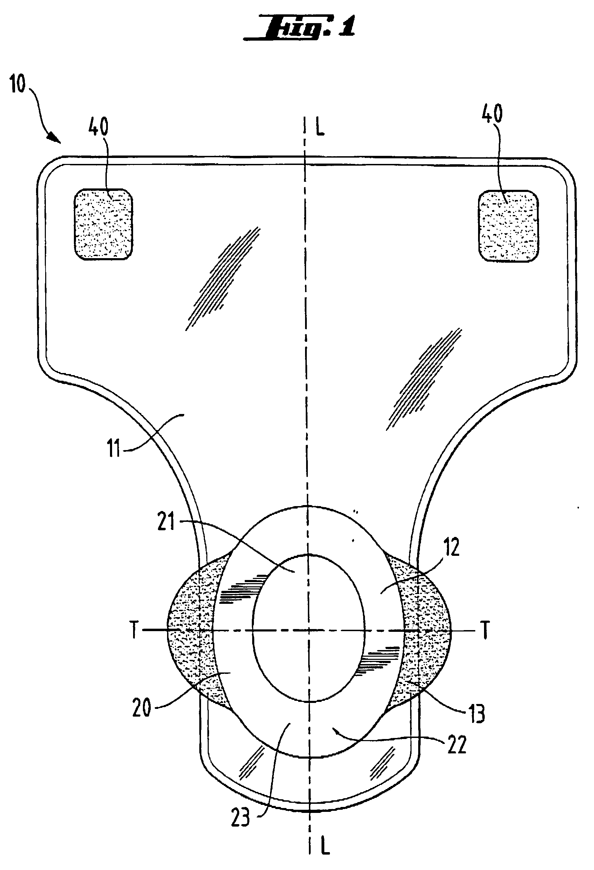

The invention relates to a urine management device (10) as shown, for example, in Figure 1. The device (10) comprises a bag (11) and a flange (12). All references to urinary management devices as made herein refer to urinary management devices which comprise a storage means such as a bag, which may also comprise absorbent material. Urinary management devices which do not have any storage capacity and typically serve to close the urethra are not within the scope of the present invention. The present invention relates to urine management device for male and for female wearers. Preferably the urine management devices are disposable.

### Description of the urine management device as a whole

The term "disposable" as used herein describes devices which generally are not intended to be laundered or otherwise restored or reused (i.e., they are intended to be discarded after a single use and, preferably, to be recycled, composted or otherwise disposed of in an environmentally compatible manner).

Referring now to Figure 1, there is shown a urine management device (10). Typically disposable urine management devices (10) comprise a bag (11) having an aperture (21) and a flange (12) surrounding the aperture (21) for preferably adhesive attachment to the uro-genital area of a wearer. Any urine management device (10) known in the art can be provided according to the present invention.

The bag (11) as used herein is a flexible receptacle for the containment of discharged urine matter. The bag (11) can be provided in any shape or size depending on the intended use thereof, i.e. whether the device is intended for bedridden patients or active patients suffering from incontinence or for infants. For example, elongated bags which are principally tubular or rectangular are typically utilised by bedridden patients and elderly incontinence sufferers. For more active wearers whether infants or adults, the urine management device should preferably be anatomically shaped such that the device follows the contours of the body and can be worn inconspicuously by the wearer under normal garments.

Particularly, preferred shapes are flat circular and flat T shaped type bags, cone shaped bags, truncated cone shaped bags and pyramidal or truncated pyramidal shaped bags. In a most preferred embodiment of the present invention, the bag (11) has a substantially flat T shape.

In addition, the bag (11) is preferably shaped to fit the uro-genital region of the wearer and ensure good contact between the flange (12) and the skin of the wearer. For example the bag (11) may be provided with a neck portion or conduit.

The bag (11) is preferably designed to provide sufficient volume for urine under a variety of wearing conditions, also when worn by a freely moving, i.e. not bedridden wearer.

The bag (11) is designed to safely contain any entrapped material, typically it will be liquid impermeable, yet it may be breathable. The bag (11) is designed of sufficient strength to withstand rupture in use, also when pressure on the bag (11) is exerted.

According to the present invention, depending on the shape of the bag (11) required, the bag (11) may be provided from a unitary piece of material or from a number of separate pieces of material, which may be identical or different and which are sealed at their respective peripheries.

According to the present invention the bag (11) can comprise one or multiple layers, preferably two or three layers. The layer on the inside of the bag, which will typically at least partially come in contact with urine and or other bodily excretions is called the inner layer. The outermost layer of the bag, which will typically at least partially come in contact with the skin to the wearer and the garments of the wearer, is called the outer layer.

The additional layers of the bag material may be provided from any material, preferably so that the bag is liquid impervious. The layers may in particular comprise any material such as nonwovens or films. In a preferred embodiment of the present invention a laminate may be formed from a nonwoven layer and a film. The laminate can be formed by means known to the man skilled in the art.

Any nonwoven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., Ill, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag is preferably provided with a hydrophobic fibrous nonwoven layer. Such material layers present an uneven surface to the skin of the wearer and thus reduce significantly the problem of occlusion and greatly improves skin healthiness.

In one preferred embodiment of the present invention the bag comprises two layers. Preferably the outer layer comprises said fibrous hydrophobic nonwoven layer and the inner layer comprises a film.

In yet another preferred embodiment of the present invention, the bag (11) comprises three layers, preferably one film and two nonwoven layers. In an even more preferable embodiment the film is interposed between the two nonwoven layers. This sequence of layers results in a closed fibrous structure, which has a particularly pleasing sensation on contact with the skin of the wearer. In yet another preferred embodiment the inner layer comprises a film and the other two layers comprise nonwovens.

Typically, the nonwoven layer is treated with a surface active material, such as a fluorchemical or other hydrophobic finishings, to provide the requisite hydrophobicity. The nonwoven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The nonwoven layer can also be treated with agents to improve the tactile perceivable softness of the bag. The agents include but are not limited to vegetable, animal or synthetic oils, silicone oils and the like. The presence of these agents are known to impart a silky or flannel-like feel to the nonwoven layer without rendering it greasy or oily to the tactile sense of the wearer. Additionally, surfactant material, including anionic, non-anionic, cationic and non-cationic surfactants, may be added to further enhance softness and surface smoothness.

Furthermore, the nonwoven layer may be impregnated with a lotion to provide desirable therapeutic or protective coating lotion benefits. The lotion is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the nonwoven layer with a solid oil phase of cream formulation or to incorporate into the nonwoven layer an array of pressure- or thermal- or hydrorupturable capsules containing for example, baby oil.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag (11) or may be secured to the inner layer of the bag (11). Any known techniques for securing absorbent material to nonwoven and film substrates may be used to secure the absorbent material to the inner layer of the bag (11). The absorbent material may also be arranged to have any desired shape or configuration (e.g., rectangular, oval, circular, etc.).

In the embodiment shown in Figures 1 the outer surface of bag (11) is provided with patches of adhesive (40) for securing the bag (11) to the body of the wearer. Preferably, the patches of adhesive (40) are positioned on the outer surface of bag (11) such that they are secured to the abdomen of the wearer in use. Any number, size and shape of adhesive patches (40) may be used depending on the intended use of the device. The adhesive (40) may be any medically approved water resistant pressure sensitive adhesive such as hydrocolloid adhesives and hydrogel adhesives. Particularly effective adhesives in providing the desired adhesive properties to secure the flange to the skin of the wearer whilst allowing for relatively painless application and removal are hydrophillic hydrogels formed from crosslinking polymers with a plastisicer to form a 3-dimensional matrix.

As shown in Figure 1 the bag (11) is provided with an aperture (21) whereby urine is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration in the longitudinal direction.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering liquids.

The flange (12) may be provided in any size depending on the wearer group for which the device is intended. Similarly the flange (12) may be provided in any shape.

The flange comprises a garment facing portion (22) and a wearer facing portion (23). In an preferred embodiment these are two large, substantially flat surfaces. In a preferred embodiment the wearer facing portion (23) of the flange (12), however, comprises a raised, curved bulge positioned beneath the aperture (21) and extending across the flange (12) for approximately the width of the aperture (21). The bulge is shaped to span the perineum of an infant. Typically urine management devices (10) for female wearers comprise such a bulge, while urine management devices (10) for male wearers do not need to comprise such a bulge.

The flange (12) should be made of soft, flexible and malleable material to allow easy placement of the flange to the uro-genital area. In addition it is preferred that the flange (12) be made of a hydrophobic material such that if urine does come into contact with the perimeter surrounding the aperture (21) it is repelled and does not wick to the outer edge of the flange (12). Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing surface (21) of the flange (12) may extend into the defined aperture area so as to form a skirt or flap of material which prevents unintentional adhesion of the surface edges of the flange (12) defining the aperture (21) to oneanother during use.

According to the present invention the urine management device further comprises an attachment means to secure the device to the wearer. Such means comprises a body-compatible pressure sensitive adhesive (20) applied to the wearer facing portion (23) of the flange (12) and may in one embodiment further comprise straps.

According to the present invention any medically approved water resistant pressure sensitive adhesive may be used to attach the device to the uro-genital area of the wearer, such as hydrocolloid adhesives and hydrogel adhesives. Particularly effective adhesives in providing the desired adhesive properties to secure the flange to the skin of the wearer at the sensitive uro-genital area, whilst allowing for relatively painless application and removal, are formed from crosslinking polymers with a plastisicer to form a 3-dimensional matrix.

Suitable adhesives for use herein are hydrogel adhesives available from 3M and Promeon.

The adhesive (20) can be applied to the wearer facing surface (22) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example for urine management devices (10) to be used for babies the amount of adhesive (20) may be less than for urine management devices designed for active adult incontinence sufferers.

According to another aspect of the present invention, the urine management device (10) has been found particularly useful and beneficial when utilized in conjunction with a garment or a disposable diaper or a faecal management device. Typically the urine management device (10) will be positioned to the uro-genital area of the wearer positioned and secured to the wearer by the adhesive flanges and patches of adhesives. Subsequently, the diaper is positioned over the urine management device and fastened in a conventional manner around the body of the wearer. It has been found that in addition to providing excellent separation between urine and faecal matter, the combined urine management device (10) and diaper system reduce skin irritation, which may at time occur, especially as the wearer group includes the very old, young and unhealthy wearers.

### Detailed description of the lobes

The invention resides principally in the provision of at least one non-adhesive lobes (13) on the flange (12).

To allow a more detailed and clear description of the device, in the following paragraphs firstly a number of terms, as used herein, will be defined.

Regarding in particular the flange (12) the longitudinal axis is to be understood as follows: The direction which is substantially defined by a line connecting the coccyx, the perineum and the genital area for a wearer with the urine management device (10) in the intended wearing position defines the longitudinal direction. The longitudinal axis is an axis in the longitudinal direction, which crosses the centre of the aperture (21). The longitudinal axis is typically also an axis of symmetry.

The transversal axis is an axis in the direction perpendicular to said longitudinal direction, which crosses said centre of said aperture (21). The transversal direction is generally aligned with the wearer's hips.

A side of the flange (12) is defined by said longitudinal axis, so that there is one side of the flange (12) on either side of said longitudinal axis.

The term contour is used with regard to flat objects, such as the flange (12), to denote the outer boundaries of the object as seen when looking perpendicularly onto the plane in which the object is flat.

The term centre is used to describe a point of an object or a part of an object, which coincides with the centre of mass, if said object or part were of uniform density.

Centred on the longitudinal or the transversal axis is used if the centre of an object lies on said longitudinal or transversal axis when looking onto the object from a direction which is perpendicular to both the longitudinal and the transversal axis.

The term upward refers to the direction along a longitudinal line which is followed when generally moving towards the head of an upright standing wearer wearing the urine management device (10) in the intended wearing position. A second object is above a first object if it can be reached by moving generally in the upward direction when starting from the first object.

The lobes (13) may comprise an integral or contiguous extension of the flange (12), or alternatively, may be made of a separate and independent piece of material joined to the flange (12). The lobes (13) have a wearer facing portion and a garment facing portion. The wearer facing portions of said lobes (13) are non-adhesive.

The term non-adhesive, as used herein, describes a low level of adhesion as compared to the parts of the flange (12) which are meant to adhere to the uro-genital area of the wearer. Preferably the adhesive forces on the non-adhesive parts are no more than 20 % than the adhesive forces for attachment to the uro-genital area of the wearer, more preferably no more than 10 % as measured by the adhesive forces test method described below. In any case the adhesive forces measured on the non-adhesive parts of the device (10) are no more than 0.5 N (50 g), preferably no more than 0.3 N (30 g) as measured by the adhesive forces test method described below. Thus, the non-adhesive lobes (13) are easier to detach from the skin in the uro-genital region and from the skin of the fingers. Such a low level of adhesion can be achieved in various ways: Preferably the non-adhesive areas are completely or largely free of adhesive. The low level of adhesion can also be achieved by covering an adhesive present on said surface to reduce adhesion, e.g. by talcum or by a solid non-adhesive layer of any kind, such as paper. If said non-adhesive layer covering said adhesive is used, said non-adhesive layer is not intended to be removed at any time by the caretaker or wearer, thus not being readily removable or releasable.

The lobes (13) extend outward from the contour of the flange (12); typically in such a direction that the wearer facing portion of the lobes (13) is in the same plane as the adjacent areas of the wearer facing portion (23) of the flange (12). In a preferred embodiment the lobes (13) are generally a convex extension of the contour of the flange (12) without lobes (13), as seen in Figures 1,2 and 3. The shape of each lobe (13) can be independently chosen. In one preferred embodiment all lobes (13) are of an identical first shape. In another preferred embodiment an even number of lobes (13), preferably two, is of an identical first shape and an even number of lobes (13), preferably two, is of an identical second shape. Such an embodiment may, for example, comprise two lobes as depicted in

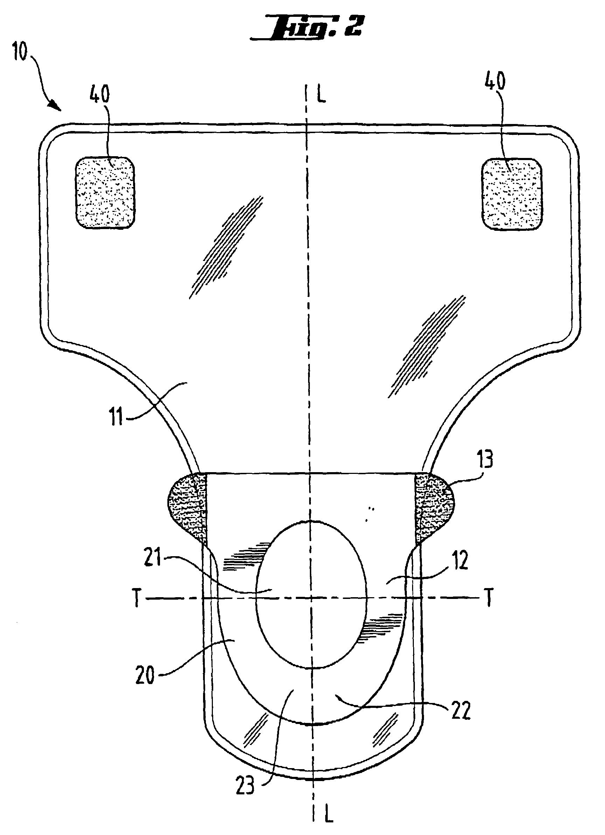

Figure 1, which are particularly helpful in placing the device (10) and two lobes, as depicted in Figure 2, which are particularly helpful in detaching the device (10).

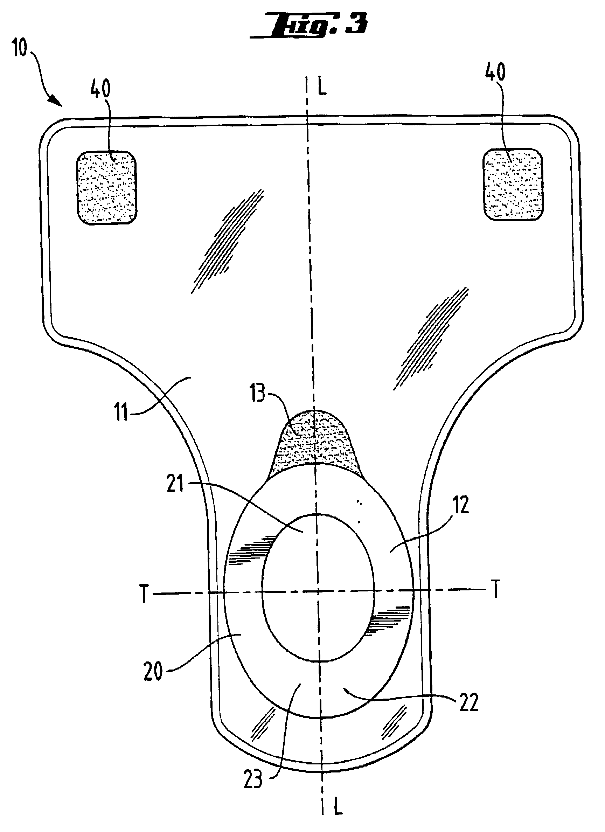

Yet another preferred embodiment is shown in Figure 3. There the urine management device (10) is provided with only one lobe at the upward oriented end of the flange (12). The lobe (13) of Figure 3 is beneficial for both attachment and detachment since it is provided in a very accessible position. Moreover, it ensures a good fit of the device for a large variety of wearers. The size of the lobes can be chosen largely independent of the width and shape of the uro-genital area of the wearer, since the lobe (13) when the urine management device (10) is worn is positioned adjacent to the abdomen of a wearer.

The size of the lobes (13) is such, that they can be gripped easily with the fingers, typically thumb and forefinger of an adult caretaker.

For the placement lobes (13) the surface area on the wearer facing portion is independently preferably from 0.25 cm² to 20 cm², more preferably from 0.5 cm² to 7 cm², even more preferably from 1 cm² to 5 cm². Preferably all placement lobes (13) have a substantially identical surface area.

The flange (12) is preferably provided with at least one lobe (13). In a preferred embodiment the same number of lobes (13) are located on each side of the flange (12). In an even more preferred embodiment the flange (12) including the lobes (13) is symmetrical about the longitudinal axis. In an even more preferred embodiment the centres of the lobes (13) on either side of the flange (12) have the same distance to the transversal axis. In the preferred embodiment of Figure 1 two lobes (13) are provided, which are centred on the transversal axis. In the preferred embodiment of Figure 2 two lobes (13) are provided, which are centred on a transversal line parallel to and above the transversal axis, the line being close to the upper end of the flange (12).

The provision of two lobes (13) and in particular their preferred positioning as described above make it particularly easy for the person handling the device to hold the device with one hand using the lobes (13) and to remove a release means, which may cover the adhesive (20), using the other hand. Moreover, the lobes (13) are a convenient help in attachment of the device (10). For example, a wearer may apply a urine management device (10) first to the perineum area and pull it upwards towards the abdomen by using the lobes (13).

Said lobes (13) can comprise any material, preferably any material comprised by the flange (12) for which typical materials are listed above. In order to improve the physical or other properties of the lobes (13), they may comprise additional material or additives or a different material composition not comprised by the flange (12), or additional layers of a material also comprised by the flange (12).

According to the present invention the lobes (13) can also be used for the detachment of the urine management device (10) from the wearer. Since the lobes (13) are non-adhesive, they are easier to grip than the flange (12) itself and furthermore avoid all the previously mentioned disadvantages associated with gripping the flange (12). The provision of at least two non-adhesive lobes (13) furthermore allows the detachment of the urine management device (10) from both sides. This is particularly desirably as a detachment from one side may be impossible due the position of the wearer, in particular in the case of a bedridden patient.

The adhesive (20) on the wearer facing portion (23) of the flange (12) is preferably covered with a release means (not shown) in order to protect the adhesive (20), such as siliconised paper. The lobes (13) are preferably also covered by the release paper. In another preferred embodiment only some of said lobes are covered with a release means. Before application of the urine management device (10) to the skin of the wearer, the release means if present is removed. Since the lobes are non-adhesive, gripping of those portions of the release means, which cover said lobes (13), is particularly easy. In another embodiment of the present invention the release means comprises a portion, preferably at least one lobe independent of the number of lobes (13), which may be provided on the flange (12), which extends beyond the contour of the flange (12).

A urine management device (10) according to the present invention will typically be placed onto the uro-genital area of a wearer by the following handling steps: Removing the release means (if present) covering the adhesive (20) of the device (10); placing said device (10) in the perineal area of the wearer; letting said adhesive (20) on said flange (12) attach to the body, namely the uro-genital area, of the wearer by holding the device (10) using the lobes (13), preferably by exercising some pressure towards the wearer, more preferably by exercising some pressure towards the uro-genital area and the abdomen of the wearer.

The detachment of a urine management device (10) according to the present invention will typically comprise the following steps: grasping with one or both hands, typically using thumb and forefinger of each hand, one or more of said lobes (13) on said flange (12); exercising forces directed substantially away from the wearer; holding the detached device (10) using said lobes on said flange (12); releasing the grasp of said lobes used for holding the detached device (10).

Depending on the used embodiment of the present invention and conditions of the placement, usage and detachment of the device (10) numerous other handling steps in placing and detaching the device (10) may also be undertaken, different handling steps may be undertaken or certain of the mentions handling steps may not be involved.

### Adhesive forces test method

### Intent:

The purpose of this method is to determine the adhesive forces due to a contact with an adhesive surface S. The method is based on the pull force required to remove a piece of cotton from the adhesive surface S.

### Test conditions:

Standard conditions are temperature of 23° ± 2° C and a relative humidity at that temperature of 50 ± 2%. All tests shall be conducted in the conditioned chamber or room under standard conditions.

The test specimen of the adhesive surface S shall be 20 ± 2 mm wide and 50 mm in length. (The test specimen may have to be provided from a material identical to the material used for said lobes, if the lobes are not large enough.)

### Equipment:

Tensile Tester:
   Instron Tester Model 6021 or equivalent - Test speed 1000 mm/min
Mechanical Weight:
   Mechanical weight driven by the tester to move 1000 mm/min in a direction and in the opposite after application of 1 Kg of mass for 30 sec on adhesive surface S.
Piece of cotton:
   The piece of cotton shall be 20 ± 2 mm wide and 50 mm in length

### Sample preparation and Execution:

Clamp the weight into the upper jaw of the adhesion tester machine. Operate the upper jaw at 1000 mm/min in down direction (compression phase) allowing the mechanical weight to match exactly the adhesive surface S covered by the piece of cotton to rest on the stationary portion of adhesion testing apparatus during the weight application period (30 s). Return the upper jaw at the same speed in the opposite direction pulling the piece of cotton and the adhesive surface S apart and use the peak pull value obtained as the pull stickiness adhesion value.

### Report:

Report the average of 2 pull peaks in N.

## Claims

1. Urine management device (10) comprising a bag (11), said bag (11) having an aperture (21) and a flange (12) surrounding said aperture (21), said flange (12) having a wearer facing portion (23) and a garment facing portion (22), wherein said wearer facing portion (23) comprises a layer of adhesive (20) for releasable attachment to the uro-genital area of a wearer,
said device (10) being characterised in that said flange (12) is provided with at least one non-adhesive lobe.

2. Urine management device (10) according to claim 1, comprising at least two lobes (13).

3. Urine management device (10) according to claim 1 comprising one lobe (13) positioned at the upward end of said flange (12).

4. Urine management device (10) according to claim 1, wherein said device (10) has a longitudinal axis of symmetry and wherein said lobes (13) are located about said axis.

5. Urine management device (10) according to any one of the preceding claims, wherein said device (10) further comprises a release means, said release means covering said adhesive (20) and said lobes (13).

6. Urine management device (10) according to any one of the preceding claims, wherein said device (10) further comprises a release means characterised in that said release means extends beyond the flange (12).

7. A method for a caretaker or a wearer for placing a urine management device (10) according to claim 1 in the uro-genital area of a wearer, said method comprises the steps of:
- placing said device (10) in the perineal area of the wearer;
- letting said adhesive (20) on said flange (12) attach to the uro-genital area of the wearer by holding said device (10) using said lobes (13);
- releasing the grasp of both of said lobes (13).

8. The method of claim 8, wherein said urine management device (10) further comprises a release means covering said adhesive (20), said method comprises a further step of removing the releasing means covering said adhesive (20) with one hand while holding the urine management device (10) using the other hand, after grasping said lobes (13).

9. A method for a caretaker or a wearer for the removal of a urine management device (10) according to claim 1, from the uro-genital area of the wearer, said method comprises the steps of:
- grasping with one or both hands one or more of said lobes (13) on said flange (12);
- exercising forces directed substantially away from the wearer by said lobes (13);
- holding the detached device (10) using said lobes (13) on said flange (12);
- releasing the grasp of said lobes (13) used for holding said detached device (10).
